# EUROPÄISCHE PATENTSCHRIFT

(11) **EP 0 918 517 B1**
(45) Veröffentlichungstag und Bekanntmachung des Hinweises auf die Patenterteilung: **28.05.2003**
(21) Anmeldenummer: 97934504.8
(22) Anmeldetag: 21.07.1997
(51) Int. Cl.: A61K 31/20, A61K 35/66, A61K 35/68, A61K 35/70, A61K 35/74, A61K 35/80, A61P 33/02

(54) **OMEGA-3-FETTSÄUREHALTIGE ZUBEREITUNG AUS MIKROORGANISMEN ALS PROPHYLAKTIKUM BZW. THERAPEUTIKUM GEGEN KOKZIDIOSE BEI TIEREN**
COMPOSITION OF MICRO-ORGANISMS CONTAINING OMEGA-3-FATTY ACID USEFUL AS A PROPHYLACTIC OR THERAPEUTIC AGENT AGAINST COCCIDIOSIS OF ANIMALS
COMPOSITION DE MICRO-ORGANISMES CONTENANT DE L'ACIDE GRAS OMEGA-3, SERVANT D'AGENT PROPHYLACTIQUE OU THERAPEUTIQUE CONTRE LA COCCIDIOSE CHEZ LES ANIMAUX

(30) Priorität: 22.07.1996 DE 19629433
(43) Veröffentlichungstag der Anmeldung: 02.06.1999
(73) Patentinhaber: Nutrinova Nutrition Specialties & Food Ingredients GmbH, 65926 Frankfurt am Main (DE)
(72) Erfinder: KIY, Thomas, D-65929 Frankfurt am Main (DE); WULLBRANDT, Dieter, D-65719 Hofheim (DE); MÜLLNER, Stefan, D-65239 Hochheim (DE); KLEIN, Ulrich, D-65779 Kelkheim (DE)
(74) Vertreter: Luderschmidt, Schüler & Partner
(86) Internationale Anmeldenummer: EP9703905
(87) Internationale Veröffentlichungsnummer: WO98003168

(56) Entgegenhaltungen:
- EP-A- 0 246 532
- WO-A-91/07498
- WO-A-93/00084
- US-A- 4 374 657
- ALLEN: "Diets hign in n-3 fatty acids reduce cecal lesion scores in chickens infected with Eimeria tenella" POULT. SCI., Bd. 75, Nr. 2, Februar 1996, Seiten 179-185, XP002047882
- DATABASE CAB ABSTR. cab international DIALOG AN = 2643290, CAB abs. an: 930801673, XP002047888 & UKOHA: "Trypanocidal action od polyunsaturated fatty acids and lysophosphatidyl-choline derivatives" 1990 , INT. LABO. RES. ANI. DIS. , NAIROBI, KENYA & Conf. Chemotherapy for typanosomiasis. Proc. workshop held at ILRAD, Nairobi, Kenya, 21-24 Aug. 1989
- DATABASE WPI Week 9204 Derwent Publications Ltd., London, GB; AN 92-027351 XP002047889 & JP 03 272 692 A (SUNTORY LTD) , 4.Dezember 1991
- PATENT ABSTRACTS OF JAPAN vol. 0, no. 0 & JP 01 215245 A (SUNTORY LTD), 29.August 1989,
- DATABASE WPI Week 8638 Derwent Publications Ltd., London, GB; AN 86-249481 XP002047890 & JP 61 177 990 A (HARIMA KASEI KOGYO KK)
- KIFFE: "Purification of docosahenaenoic acid (DHA) produced by marine microalgae Isochrysis galbana" J. MARINE BIOTECHNOL., Bd. 2, Nr. 3, 1995, Seiten 139-142, XP002047883
- LOPEZ: "Improvement of eicosapentaenoic acid content in isolates of Isochrysis galbana" J. MARINE BIOTECHNOL., Bd. 1, Nr. 3, 1993, Seiten 147-149, XP002047884
- TAKEYAMA: "DHA enrichment of rotifiers : a simple two-step culture using the unicellular algae Chlorella regularis and Isochrysis galbana" J. MARINE BIOTECHNOL., Bd. 3, Nr. 4, 1996, Seiten 244-247, XP002047885
- ALLEN: "Interaction of dietary flaxseed with coccidial infections in chickens" POULTR. SCI., Bd. 76, Nr. 6, Juni 1997, Seiten 822-827, XP002047886
- PATENT ABSTRACTS OF JAPAN vol. 0, no. 0 & JP 08 133980 A (HARIMA CHEM. IND.), 28.Mai 1996,
- KENDRICK: "Lipids of selected molds grown for production of n-3 and n-6 polyunsaturated fatty acids" LIPIDS, Bd. 27, Nr. 1, Januar 1992, Seiten 15-20, XP002047887
- PATENT ABSTRACTS OF JAPAN vol. 0, no. 0 & JP 04 088954 A (SAGAMI CHRM. RES. CENTER)

## Beschreibung

Die vorliegende Erfindung betrifft die Verwendung einer omega-3-fettsäurehaltigen Zubereitung aus aquatischen Mikroorganismen in form von Biomasse und/oder in Form eines Öls zur Herstellung eines Arzneimittels zur prophylaktischen und therapeutischen Anwendung gegen die Kokzidiose bei Tieren.

Omega-3-Fettsäuren (Omega-3-Polyunsaturated fatty acids = Omega-3-PUFAs) - insbesondere EPA (Eicosapentaensäure) und DHA (Docosahexaensäure) - gewinnen in letzter Zeit für die menschliche Emährung immer mehr an Bedeutung. Sie weisen positive diätetische Effekte auf und haben eine gesundheitsfördernde Wirkung. Die Anreicherung der menschlichen Nahrung mit Omega-3-Fettsäuren ist im Hinblick auf eine Senkung des Cholesterinspiegels sowie eine Reduktion der Häufigkeit von Herz-Kreislauferkrankungen sinnvoll. In einer Vielzahl von Studien wurde der positive Einfluß von Omega-3-PUFAs auf pathologische Stoffwechselprozesse belegt. Die Docosahexaensäure spielt außerdem als Baustein im zentralen Nervensystem (Gehirn) sowie in der Netzhaut eine wichtige Rolle.

Relativ wenig Untersuchungen, mit Ausnahme der Fischzucht, liegen bisher hinsichtlich der Bedeutung der Omega-3-PUFAs im Bereich der Tiergesundheit vor. Omega-3-PUFA-Präparate wurden bisher primär über das Futter an die Tiere mit dem Ziel verabreicht, die für den menschlichen Verzehr bestimmten tierischen Produkte (Eier, Fleisch) mit Omega-3-PUFAs anzureichern. So konnte der Omega-3-PUFA Gehalt im Fettgewebe der Muskeln von Masthähnchen durch Verfüttern eines PUFA-haltigen Produkts signifikant gesteigert werden (Mooney & van Elswyk, 1995, Poultry Science 74, Suppl. 1, 89). Weiterhin konnte gezeigt werden, daß durch Anreicherung des Legehennenfutters mit Omega-3-PUFA-haltigen Komponenten (Leinöl, Rapsöl, Sojaöl, Fischöl) eine Anreicherung mit Omega-3-PUFAs im Ei möglich ist (Herber & van Elswyk, 1995, Poultry Science 74, Suppl. 1, S. 57 ; Grashorn und Blanch 1996, DGS 6 , 6 - 9).

Über den Einsatz von Omega-3-PUFA-Präparaten im Rahmen von prophylaktischen sowie therapeutischen Maßnahmen zur Bekämpfung parasitärer Erkrankungen bei Nutztieren liegen bisher keine Untersuchungen vor. In der modernen Geflügelhaltung hat die durch eine bestimmte Protozoenspezies (Gattung Eimeria) hervorgerufene Kokzidiose als intestinale Erkrankung aufgrund der hohen Verlustquoten für die Praxis eine große wirtschaftliche Bedeutung. In einer kürzlich veröffentlichten Studie konnte gezeigt werden, daß durch Anreicherung des Futters mit Fischöl das Kokzidieninfektionsgeschehen positiv beeinflußt ( Senkung der Anzahl/Intensität der Läsionen im Intestinum, Reduktion der Gewichtsverluste) werden kann (Muirhead, 1995, Feedstuffs, November 6 1995, 12). Dieser Effekt wurde auf die Wirkung der im Fischöl vorhandenen Omega-3-PUFAs zurückgeführt.

Die Verwendung von Fischöl als Quelle für Omega-3-Fettsäuren ist jedoch nicht unproblematisch, da neben einer Reihe weiterer Fettsäuren auch Cholesterin und bestimmte Schwermetalle im Fischöl vorkommen. Außerdem schwankt die Zusammensetzung der PUFA-Gehalte je nach Fischart, Jahreszeit und den Fanggründen zum Teil erheblich, wodurch sich die Herstellung von in ihren Konzentrationsgehalten einheitlichen PUFA-Produkten aus Fisch als schwierig erweist. Weiterhin kann Fischöl aufgrund der Geschmacks- und Geruchsbeeinflussung des Endproduktes nur in einer begrenzten Menge in den Futterdiäten eingesetzt werden. Als weiteres Problem ist hervorzuheben, daß die Konzentration an Omega-3-PUFAs im Fischöl in der Regel bei maximal 15 - 20% liegt und eine für die Herstellung eines Futterzusatzes notwendige Konzentrierung der Fettsäuren technisch äußerst schwierig ist.

Vorliegende Erfindung löst die vorgenannten Probleme durch die Verwendung von aquatischen Mikroorganismen (Mikroalgen, Protozoen, Pilze, Bakterien) als Produktionsstämme zur Herstellung von Zubereitungen, welche Omega-3-PUFAs, insbesondere Eicosapentaensäure (EPA) und Docosahexaensäure (DHA), enthalten. Diese Zubereitungen werden als Futteradditiva eingesetzt um ihre antiparasitische (insbesodere kokzidiostatische Wirkung) zu entfalten. Die Produzentenstämme werden heterotroph oder autotroph kultiviert, geerntet und anschließend getrocknet und/oder extrahiert. Die PUFA-Zubereitungen können dabei in Form von Biomasse oder Öl als Futteradditivum eingesetzt werden.

Demgemäß betrifft die vorliegende Erfindung im einzelnen die Verwendung einer omega-3-fettsäurehaltigen Zubereitung, erhältlich aus aquatischen Mikroorganismen zur Herstellung eines Arzneimittels zur prophylaktischen oder therapeutischen Anwendung gegen die Kokzidiose bei Tieren.

Die Zubereitung ist dadurch gekennzeichnet, daß sie aus der durch Kultivierung von aquatischen Mikroorganismen erhältlichen Biomasse besteht, in Form eines Öls vorliegt oder beidem.

Gemäß einer besonders bevorzugten Ausführungsform der vorliegenden Erfindung ist die omega-3-fettsäurehaltige Zubereitung erhältlich aus heterotroph kultivierbaren Mikroalgen, wobei diese besonders bevorzugt zur Gattung Crypthecodinium oder zur Gattung Euglena gehören.

In einer weiteren bevorzugten Ausführungsform ist die genannte Zubereitung aus niederen Pilzen erhältlich, vorzugswelse der Gattungen Thraustochytrium oder Mortierella.

In einer weiteren bevorzugten Ausgestaltung der vorliegenden Erfindung gehören die aquatischen Mikroorganismen zu den photosynthetisierenden Mikroalgen, vorzugsweise dadurch gekennzeichnet, daß diese ausgewählt sind aus den Gattungen Phaeodactylum, Isochrysis, Monodus, Porphyridium, Spirulina, Chlorella, Botryococcus, Cyclotella, Nitzschia, Dunaliella und Nannochloropsis. Eine weitere bevorzugte Ausgestaltung sieht vor, daß die genannte Zubereitung aus Bakterien erhältlich ist, vorzugsweise ausgewählt aus den Gattungen Alteromonas und Shewanella.

Die anliegende Figur, die Bestandteil dieser Anmeldung ist, enthält die graphische Auswertung der Oozystenausscheidung von Hähnchen einer Kontrollgruppe ohne Verabreichung von PUFA sowie einer Gruppe, der PUFA verabreicht wurde.

Wahlweise ist die Zubereitung aus Mischungen zweier oder mehrerer verschiedener der genannten aquatischen Mikroorganismen erhältlich.

Die omega-3-fettsäurehaltige Zubereitung ist besonders geeignet zur Herstellung eines Arzneimittels zur prophylatischen und therapeutischen Anwendung gegen die Kokzidiose des Geflügels.

Dementsprechend betrifft die vorliegende Erfindung auch ein Arzneimittel zur prophylaktischen oder therapeutischen Anwendung gegen die Kokzidiose bei Tieren, enthaltend eine omega-3-fettsäurehaltige Zubereitung, die wie vorgehend beschrieben, erhältlich ist.

Das Arzneimittel kann auch mit einem oder mehreren weiteren Arzneistoffen kombiniert werden.

Das erfindungsgemäße Arzneimittel zur Therapie und Prophylaxe der Kokzidiose ist besonders wirksam, wenn es mindestens ein weiteres Kokzidiostatikum, vorzugsweise Salinomycin enthält.

Im folgenden wird die vorliegende Erfindung im einzelnen beschrieben.

Als Produzenten zur Herstellung der ω-3-fettsäurehaltigen Zubereitung eignen sich besonders heterotroph wachsende Mikroalgen und Protozoen wie Crypthecodinium und Euglena; niedere Pilze wie Thraustochytrium, Schizochytrium und Mortierella; photosynthetisierende Mikroalgen der Gattungen Phaeadactylum, Isochrysis, Monodus, Porphyridium, Spirulina, Chlorella, Botryococcus, Cyclotella, Nitzschia, Dunaliella und Nannochloropsis und Bakterien etwa der Gattungen Alteromonas und Shewanella.

Das Herstellungsverfahren der ω-3-fettsäurehaltigen Zubereitung beinhaltet folgende Schritte:
- Animpfen des Kulturmediums mit dem Produzentenstamm.
- Inkubation der Kultur unter geeigneten Bedingungen, wobei die Kultivierung als batch-, fed-batch- oder kontinuierliche Fermentation ausgelegt sein kann.
- Ernte der Kultur, wobei u. a. folgende Methoden verwendet werden können: Cryopelletierung, Filtration, Zentrifugation, Sprühtrocknung.
- Wird nicht die Biomasse direkt als Omega-3-PUFA-Zubereitung verwendet, kann sich ein Extraktionsschritt (z. B. überkritische CO₂-Extraktion oder Extraktion mit organischen Lösungsmitteln) zur Gewinnung eines Extrakts anschließen, wobei entweder die feuchte oder die getrocknete Biomasse eingesetzt wird.

Optional kann die als Extrakt vorliegender Zubereitung vor der Verwendung gereinigt werden, z. B. über SFC (supercritical fluid chromatography) oder HPLC-Methoden.

Die Omega-3-PUFAS können in der erfindungsgemäßen Zubereitung u. a. in Form von Phospholipiden, Glycolipiden, Mono-, Di- oder Triglyceriden oder Sulpholipiden oder aber als freie Säuren bzw deren Ethylester vorliegen.

Der DHA- bzw. EPA-Gehalt der dem Futter zugemischten Omega-3-PUFA-Zubereitung (Biomasse oder Öl) liegt zwischen 5 und 80% bezogen auf den Gesamtfettsäuregehalt.

Weiterhin kann das Öl vor der Verwendung mikroverkapselt werden, um eine bessere Verarbeitung und einen Schutz der PUFAs zu gewährleisten. Zur Erhöhung der oxidativen Stabilität der Omega-3-PUFAs können der Zubereitung Stabilisatoren wie etwa Tocopherol und Ascorbylpalmitat zugeführt werden.

Die kokzidiostatische Wirkung der omega-3-fettsäurehaltigen Zubereitung wird im Rahmen von Infektionsversuchen bei der Zieltierart Masthähnchen überprüft. Als Infektionsspezies werden Eimeria acervulina, E. maxima und E. tenella miteinbezogen. Die Versuche laufen über einen Zeitraum von 3 Wochen. In den Versuchen wird die Wirkung der PUFAs auf das Infektionsgeschehen in zwei verschiedenen Konzentrationen überprüft.

### Applikation und Behandlungsplan:

| Behandlungsgruppen | | Zubereitung | Tierzahl | Dosierung | Infektion |
|---|---|---|---|---|---|
| A | Kontrolle | - | 15 | - | - |
| B | Behandlung | ja | 15 | Dosis 1 | - |
| C | Behandlung | ja | 15 | Dosis 2 | - |
| D | Kontrolle | - | 15 | - | ja |
| E | Behandlung | ja | 15 | Dosis 1 | ja |
| F | Behandlung | ja | 15 | Dosis 2 | ja |

Während der Untersuchungen werden verschiedene Meßparameter erfaßt. Folgende Parameter geben Auskunft über die Wirksamkeit der PUFAs:
- individuelle Gewichtsentwicklung der Tiere
- Oozystenausscheidung
- Reduktion der Lesionen im Darm (Erfassung nach lesion score index)
- Mortalitätsrate

Weiterhin kann im Rahmen von Sensitivitätstests die Wirkung des Kokzidiostatikums Salinomycin in Kombination mit der ω-3-fettsäurehaltigen Zubereitung gemäß der vorliegenden Erfindung überprüft werden.

Die Schädigung adulter Würmer (therapeutischer Effekt) und sich entwickelnder Wurmstadien (prophylaktischer Effekt) in einem die erfindungsgemäße Zubereitung enthaltenden Milieu kann in geeigneten in vitro Testmodellen mit nicht parasitisch lebenden Würmern (z. B. Caenorhabditis elegans) oder mit exogenen, sich in der Außenwelt entwickelnden Stadien parasitischer Würmer (z. B. Trichostrongyliden der Wiederkäuer) geprüft werden. Als Kriterien für die Wirksamkeit der erfindungsgemäßen Zubereitung werden Überleben und Beweglichkeit der Würmer sowie die Entwicklungsrate der juvenilen Stadien herangezogen.

### Versuch:

Untersucht wurde der Einfluß der Kombination PUFA (Docosahexaensäure aus Crypthecodinium) und Salinomycin auf das Infektionsgeschehen nach Infektion mit E. tenella (Houghton, Lab.-Stamm) unter besonderer Berücksichtigung der individuellen Gesamtoozystenauscheidung im Rahmen eines Fütterungsversuches bei Masthähnchen.

In dem Versuch wurde bei Fütterung einer maisbetonten Diät (Maisanteil 44,6 %) die Wirkung der Kombination Docosahexaensäure/Salinomycin auf die Eimeriainfektion untersucht. Um eine Aussage über den Einfluß der Kombination auf die Pathogenität des Erregers treffen zu können, wurde die Gesamtoozystenauscheidung sowie die Ausprägung der Lesion scores in den Caeca jedes Versuchstiers festgestellt. Die Gewjcntsentwicklung und Futterverwertung der Tiere gaben weitere Hinweise auf die Ausprägung des Infektionsgeschehens.

### Kenndaten der Prüfung

| | | |
|---|---|---|
| Versuchszeitraum | 20 Tage | |

| Prüfpräparate | | |
|---|---|---|
| Name | Sacox® | RBD-DHASCO® Docosahexaensäure |
| Konzentration | Wirkstoff | enthält 40 % Docosahexaensäure |
| | Salinomycin | (DHA) |
| Hersteller | HRVET | Martek Biosciences Corporation, USA |
| | | |

| Prüfsystem | | |
|---|---|---|
| Spezies | Lohmann-Mastküken | |
| Geschlecht | männlich | |
| Alter | Eintagsküken | |

### Versuchsphasen

| Versuchsphase | Versuchstag |
|---|---|
| Ankunft der Tiere / Versuchsbeginn Gruppeneinteilung | d - 8 |
| Gruppenkotkontrolle auf Fremdinfektionen Kennzeichnung der Tiere (Geflügelmarken) Beginn der Einzelhaltung | d - 3 |
| Infektion der Tiere (Challenge) | d0 |
| Gruppenkotkontrolle auf Fremdinfektionen | d+3 |
| Gruppenkotsammlung (Kontrollgruppen) Individuelle Kotsammlung (Infektionsgruppen) Bestimmund des total oocyst output | d+4 bis d+10 |
| Bestimmung des Lesion score | d+11 |

### Applikation und Behandlungsschema

| | |
|---|---|
| Applikation von Sacox® + RBD-DHASCO® | oral mit dem Futter |
| Infektion mit E. tenella, Houghton (Lab. Stamm) | oral mit der Schlundsonde |
| Infektionsdosis | 500 versporte Oozystenl Tier (in 1 ml 1 % w/v wässriger Ochsengallelösung) |
| Alter der Oozysten bei Infektion | 21 Tage |

| Gruppen | Bezeichnung/Futterration | Präparat | n | Dosierung | Infektion |
|---|---|---|---|---|---|
| A | Kontrolle / Maisdiät | - | 15 | - | - |
| B | Sacox | Salinomycin | 15 | 60 ppm | - |
| C | Sacox/RBD-DHASCO | Salin./DHA | 15 | 60 ppm/ 4% | - |
| D | Kontrolle / Maisdiät | - | 15 | - | ja |
| E | Sacox | Salinomycin | 15 | 60 ppm | ja |
| F | Sacox/RBD-DHASCO | Salin./DHA | 15 | 60 ppm/ 4% | ja |

### Aufarbeitung des Probenmaterials:

### Kotsammlung:

Der Kot wurde täglich über einen Zeitraum von 24 Stunden in Kunststorfschalen, die mit 4 %iger Kaliumdichromat-Lösung versetzt waren, gesammelt. Die gesamte Kotmenge wurde in ein Sammelgefäß überführt.

### Aufarbeitung der Kotproben:

Die Einzelkotproben wurden gerührt (Magnetrührer, 250 rpm), gesiebt (Maschenweite 1 mm) und mit konz. NaCl-Lösung auf 300 oder 800 ml Endvolumen (abhängig von der zu erwarteten Oozystenausscheidung) eingestellt. Im Anschluß wurde kristallines NaCI unter Rühren im Überschuß zugegeben. Während des Rührens wurde ca. 1 ml der Suspension mit einer Pipette oder einer Spritze aufgezogen (mittlerer Bereich der Flüssigkeitssäule) und damit die McMaster-Kammer gefüllt.

### Auszählung der Oozysten:

Die Kammern wurden von der dem Untersucher zugewandten Seite blasenfrei gefüllt. Vor der Beschickung wurde das Deckglas so verschoben, daß sich ein ca. 3 mm breiter Spalt über den Kammern bildet. Vor der Zählung wurde das Deckglas in die eigentliche Position gebracht (Kammern müssen vollständig abgedeckt sein). Nach ca. 5 minütiger Flotation wurden die Oozysten in den 10 Abschnitten der 3 Zählnetze bei 80 oder 320 facher Vergrößerung ausgezählt.

Unter Berücksichtigung des Volumens unter jedem Zählnetz (10 mm x 10 mm x 1,5 mm = 0,15 ml) und des Volumens der Suspension, wurde die Oozystenzahl berechnet.

### Versuchsdurchführung:

Der gesamte Versuch wurde nach dem Prinzip der Guten Labor Praxis durchgeführt.

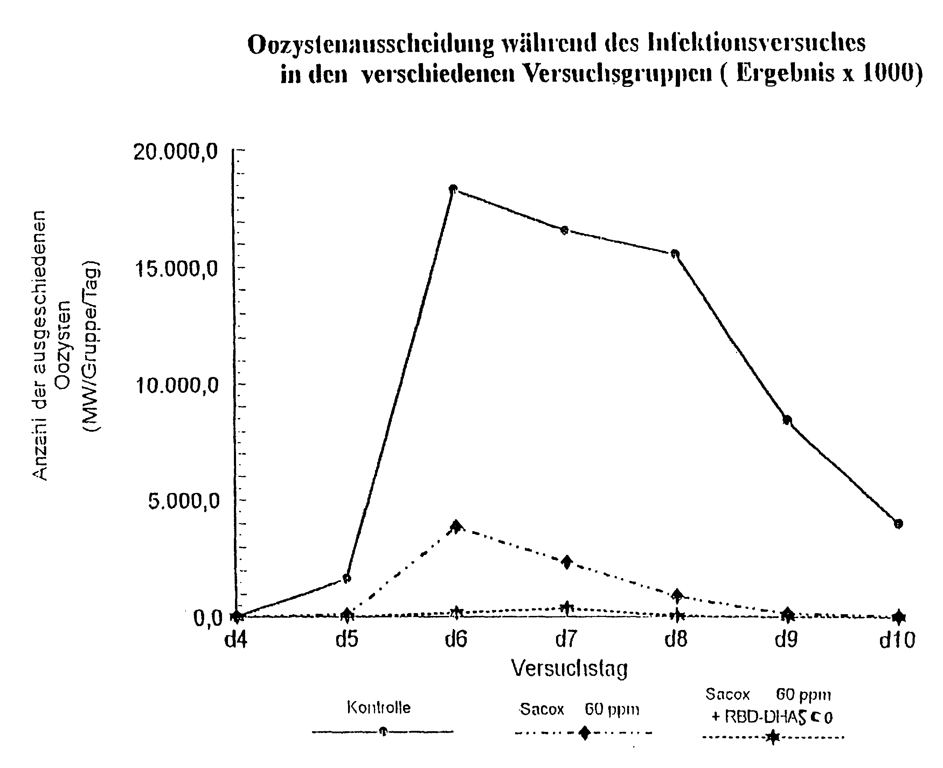

Die Ergebnisse sind in der nachfolgenden Tabelle sowie der Figur dargestellt.

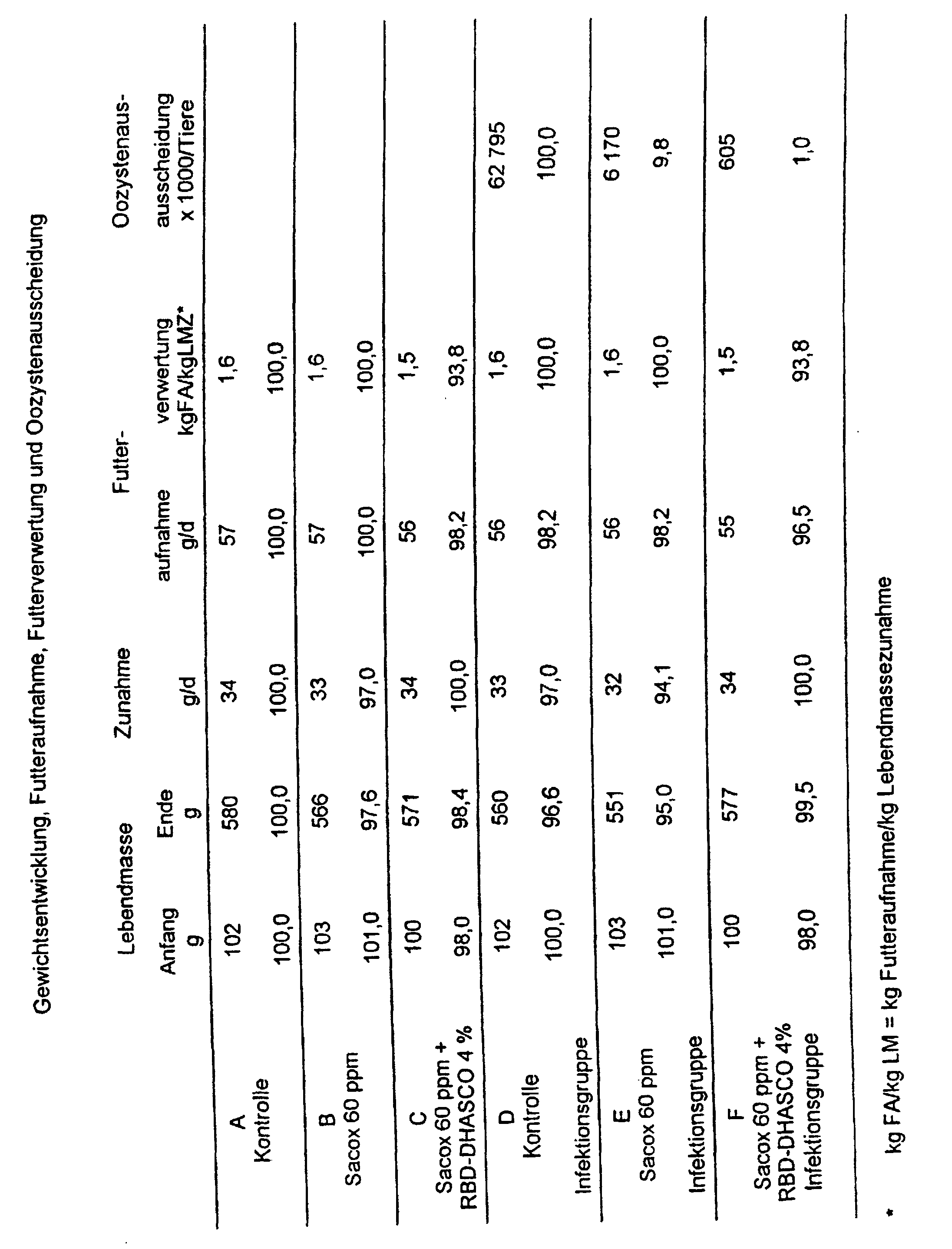

## Patentansprüche

1. Verwendung einer omega-3-fettsäurehaltigen Zubereitung aus aquatischen Mikroorganismen in Form von Biomasse und/oder in Form eines Öls zur Herstellung eines Arzneimittels zur prophylaktischen oder therapeutischen Anwendung gegen die Kokzidiose bei Tieren.

2. Verwendung nach Anspruch 1, **dadurch gekennzeichnet, daß** die Zubereitung 40% DHA enthält.

3. Verwendung nach Anspruch 1 oder Anspruch 2, **dadurch gekennzeichnet, daß** die Mikroorganismen heterotroph kultivierbare Mikroalgen sind.

4. Verwendung nach Anspruch 3, **dadurch gekennzeichnet, daß** die Mikroalgen zur Gattung *Crypthecodinium* gehören.

5. Verwendung nach Anspruch 3, **dadurch gekennzeichnet, daß** die Mikroalgen zur Gattung *Euglena* gehören.

6. Verwendung nach Anspruch 1 oder Anspruch 2, **dadurch gekennzeichnet, daß** die Mikroorganismen niedere Pilze sind.

7. Verwendung nach Anspruch 6, **dadurch gekennzeichnet, daß** die niederen Pilze zur Gattung *Thraustochytrium* gehören.

8. Verwendung nach Anspruch 6, **dadurch gekennzeichnet, daß** die niederen Pilze zur Gattung *Mortierella* gehören.

9. Verwendung nach Anspruch 1 oder Anspruch 2, **dadurch gekennzeichnet, daß** die Mikroorganismen zu den photosynthetisierenden Mikroalgen gehören.

10. Verwendung nach Anspruch 9, **dadurch gekennzeichnet, daß** die Mikroalgen ausgewählt sind aus den Gattungen *Phaedactylum, Isochrysis, Monodus, Porphyridium, Spirulina, Chlorella, Botryococcus, Cyclotella, Nitzschia, Dunaliella* und *Nannochloropsis.*

11. Verwendung nach Anspruch 1 oder Anspruch 2, **dadurch gekennzeichnet, daß** die Mikroorganismen zu den Bakterien gehören.

12. Verwendung nach Anspruch 11, **dadurch gekennzeichnet, daß** die Bakterien ausgewählt sind aus den Gattungen *Alteromonas* und *Schewanella.*

13. Verwendung nach Anspruch 1 oder Anspruch 2, **dadurch gekennzeichnet, daß** die Zubereitung aus Mischungen zweier oder mehrerer verschiedener Mikroorganismen gemäß einem der Ansprüche 3 bis 12 erhältlich ist

14. Verwendung nach einem der vorstehenden Ansprüche, **dadurch gekennzeichnet, daß** die Kokzidiose bei Tieren die Kokzidiose des Geflügels ist.

## Claims

1. Use of a preparation containing omega-3-fatty acid made from aquatic micro-organisms in the form of biomass and /or in the form of an oil to prepare a drug for prophylactic or therapeutic use to treat coccidiosis in animals.

2. Use in accordance with claim 1, **characterised in that** the preparation contains 40 % DHA.

3. Use in accordance with claim 1 or claim 2 **characterised in that** the micro-organisms are micro-algae capable of heterotrophic cultivation.

4. Use in accordance with claim 3, **characterised in that** the micro-algae belong to the species *Crypthecodonium*.

5. Use in accordance with claim 3, **characterised in that** the micro-algae belong to the species *Euglena*.

6. Use in accordance with claim 1 or claim 2, **characterised in that** the micro-organisms are lower fungi.

7. Use in accordance with claim 6, **characterised in that** the lower fungi belong to the species *Thraustochytrium*.

8. Use in accordance with claim 6, **characterised in that** the lower fungi belong to the species *Mortierella*.

9. Use in accordance with claim 1 or claim 2, **characterised in that** the micro-organisms belong to the photosythesising micro-algae.

10. Use in accordance with claim 9, **characterised in that** the micro-algae are selected from the species *Phaedactylum, Isochrysis, Monodus, Porphyridium, Spirulina, Chlorella, Botrococcus, Cyclotella, Nitzschia, Dunaliella* and *Nannochloropsis*.

11. Use in accordance with claim 1 or claim 2, **characterised in that** the micro-organisms belong to the bacteria.

12. Use in accordance with claim 11, **characterised in that** the bacteria are selected from the species *Alteromonas* and *Schewanella*.

13. Use in accordance with claim 1 or claim 2, **characterised in that** the preparation can be obtained from mixtures of two or more different micro-organisms in accordance with one of the claims 3 -12.

14. Use in accordance with one of the previous claims, **characterised in that** the coccidiosis occurring in animals is the coccidiosis associated with poultry.

## Revendications

1. Utilisation d'une composition contenant de l'acide gras oméga-3 de micro-organismes aquatiques sous forme de biomasse et/ou sous forme d'une huile, pour la préparation d'un médicament pour l'utilisation prophylactique ou thérapeutique contre la coccidiose chez les animaux.

2. Utilisation selon la revendication 1, **caractérisée en ce que** la composition contient 40 % de DHA.

3. Utilisation selon la revendication 1 ou 2, **caractérisée en ce que** les micro-organismes sont des micro-algues hétérotrophes cultivables.

4. Utilisation selon la revendication 3, **caractérisée en ce que** les micro-algues appartiennent au genre *Crypthecodinium*.

5. Utilisation selon la revendication 3, **caractérisée en ce que** les micro-algues appartiennent au genre *Euglena.*

6. Utilisation selon la revendication 1 ou 2, **caractérisée en ce que** les micro-organismes sont des champignons inférieurs.

7. Utilisation selon la revendication 6, **caractérisée en ce que** les champignons inférieurs appartiennent au genre *Thraustochytrium.*

8. Utilisation selon la revendication 6, **caractérisée en ce que** les champignons inférieurs appartiennent au genre *Mortierella.*

9. Utilisation selon la revendication 1 ou 2, **caractérisée en ce que** les micro-organismes appartiennent aux micro-algues photosynthétiques.

10. Utilisation selon la revendication 9, **caractérisée en ce que** les micro-algues sont choisies parmi les genres *Phaedactylum, Isochrysis, Monodus, Porphyridium, Spirulina, Chlorella, Botryococcus, Cyclotella, Nitzschia, Dunaliella* et *Nannochloropsis.*

11. Utilisation selon la revendication 1 ou 2, **caractérisée en ce que** les micro-organismes appartiennent aux bactéries.

12. Utilisation selon la revendication 11, **caractérisée en ce que** les bactéries sont choisies parmi les genres *Alteromonas* et *Schewanella.*

13. Utilisation selon la revendication 1 ou 2, **caractérisée en ce que** la composition est obtenue à partir de mélanges de deux ou plusieurs micro-organismes différents selon l'une des revendications 3 à 12.

14. Utilisation selon l'une des revendications précédentes, **caractérisée en ce que** la coccidiose chez les animaux est la coccidiose de la volaille.
